# EUROPEAN PATENT SPECIFICATION

(11) **EP 4 284 294 B1**
(45) Date of publication and mention of the grant of the patent: **11.06.2025**
(21) Application number: 22702779.4
(22) Date of filing: 31.01.2022
(51) Int. Cl.: A61C 8/00

(54) **A NOZZLE FOR A SYRINGE AND A SYRINGE WITH A NOZZLE FOR DISTRIBUTING BLOOD PLASMA AT A TARGET TREATMENT SITE WITHIN A PATIENT'S MOUTH**
DÜSE FÜR EINE SPRITZE UND EINE SPRITZE MIT EINER DÜSE ZUM VERTEILEN VON BLUTPLASMA AN EINER ZIELBEHANDLUNGSSTELLE IM MUND EINES PATIENTEN
EMBOUT POUR SERINGUE ET SERINGUE AVEC EMBOUT POUR DISTRIBUER DU PLASMA SANGUIN SUR UN SITE DE TRAITEMENT CIBLE DANS LA BOUCHE D'UN PATIENT

(30) Priority: 29.01.2021 GB 202101240; 29.01.2021 GB 202101290
(43) Date of publication of application: 06.12.2023
(73) Proprietor: NaturalSkn Limited, East Kilbride South Lanarkshire Glasgow G75 8TR (GB)
(72) Inventor: GRAHAM, Callum, Glasgow Central Scotland G75 8TR (GB)
(74) Representative: Cameron Intellectual Property Ltd
(86) International application number: PCT/GB2022/050258
(87) International publication number: WO 2022/162402

(56) References cited:
- KR-A- 20100 074 860
- KR-B1- 102 282 077

## Description

### TECHNICAL FIELD

The present disclosure relates to a nozzle for a syringe and in particular, but not exclusively, to a nozzle for applying platelet rich blood plasma to a target treatment site within a patient's maxillary sinus for bone augmentation and a syringe for distributing blood plasma at a target treatment site within a patient's mouth.

### BACKGROUND

Personalised Regenerative Medicine is the phrase that describes the current trend towards using natural, non-animal products to harness and accentuate the body's natural healing process. These biomedical technologies are aimed at stimulating tissue regeneration by applying autologous proteins to a target treatment site.

In oral surgery and implantology one commonly used way of doing this is with the use of Platelet-Rich Plasma (PRP). This technique involves taking a sample of the patient's blood, centrifuging it to separate the base constituents (red blood cells, leukocytes and plasma) into layers and extracting the blood plasma with the associated platelets and proteins.

The platelet rich blood plasma extracted from the patient's blood may be injected to a target treatment site within the patient's mouth to promote bone growth or augmentation. A dental implant may subsequently be fitted to the augmented bone.

However, the current method of maxillary sinus bone augmentation procedures is a much more invasive procedure that involves using animal products to promote bone growth and healing. These animal products can be of bovine, porcine or equine origin. Injecting platelet rich blood plasma to a target treatment site is a less invasive procedure and there is a lower risk of the patient's delicate maxillary sinus lining rupturing or bursting when the blood plasma is injected at the target site. There is also a much reduced chance of the patient having an adverse or allergic reaction to the blood plasma treatment when compared to the use of animal products. The PRP treatment is 100% autologous.

To inject the animal derived bone augmentation materials to a to a target treatment site the dental surgeon initially anaesthetises the patient in the region of the target site. The dental surgeon may then gain access to the maxillary sinus using a lateral window approach. The dentist raises a larger full thickness mucosal flap, 6 to 10 centimetres long in the patient's gum as the access point to the target treatment site within the maxillary sinus. An access hole is then made through the bone of the anterior wall of the maxillary sinus to allow the surgeon to deliver bone augmentation materials to target site.

This access window can be up to 15 x 30 mm in dimension. Care must be taken not to perforate the delicate Schneiderian Membrane which lines the interior surface of the sinus. There is a need for apparatus that allows the access window to be created at the target site without the risk of rupturing or damaging the patient's Schneiderian Membrane. The Schneiderian membrane is then detached from the walls and floor of the sinus using specialised metal instruments and working through the access window. If the membrane ruptures it may be necessary to abandon the procedure and repeat it with a second attempt, after a suitable healing period, usually 3-6 months. The bone augmentation material is then spooned or syringed into the sinus, In the area of required augmentation, between the patient's bone and Schneiderian membrane. At this stage the surgeon must again take care not to perforate the membrane of the sinus.

The lateral window approach is an invasive procedure which involves lifting a surgical flap of the patient's gum and creating an access window through the patient's bone to gain access to the target site for bone augmentation within the patient's sinus cavity. Furthermore, due to fragile nature of the sinus membrane the dentist must take extreme care not to rupture the sinus lining when separating the membrane from the bone and when delivering the bone substitute material to the target site. This is a challenging procedure with a high risk of failure, infection or morbidity for the patient.

There is therefore a need for a less invasive procedure to target the delivery of a biocompatible substrate that will induce the formation of the patient's bone, within the patient's sinus cavity. The substrate is Platelet Rich Plasma, obtained from a sample of blood from the patient that is centrifuged and processed. This separates the blood plasma from the remaining blood constituents. The separated plasma is heated in an oven to prepare the PRP gel in accordance with the standard treatment protocol for the PRP gel. Furthermore, there is a need for apparatus that allows the blood plasma to be delivered to the target site without the risk of rupturing or damaging the patient's sinus membrane.

KR 2010 0074860 A describes an apparatus and method for lifting the maxillary sinus mucous membrane during liquid injection.

It is an aim of the present invention to address one or more of the disadvantages associated with the prior art.

### SUMMARY OF THE INVENTION

In an aspect of the present invention, there is provided a nozzle for a syringe for distributing blood plasma at a target treatment site within a patient's mouth, the nozzle comprising: a body having a passage extending through the body along a longitudinal axis of the body, the passage extending from a proximal end to a distal end of the body; an inlet to the passage at the proximal end for receiving blood plasma from the syringe; an outlet from the passage at the distal end for distributing blood plasma at the target treatment site; wherein the outlet comprises an outlet aperture located on a distal tip of the nozzle; and at least one exit gate extending from the passage through the distal end of the body, wherein the at least one exit gate tapers outwardly from the passage.

The nozzle beneficially allows platelet rich blood plasma to be distributed at a target treatment site without damaging or tearing the maxillary sinus lining of a patient. This may be achieved by promoting distribution of the blood plasma at the target treatment site through a plurality of exit gates and an outlet aperture at the distal tip of the nozzle. The outlet aperture may be relatively narrow, for example about 1mm in diameter which minimises the flow of blood plasma that may be in direct contact with the sinus lining.

Furthermore, the at least one exit gate may extend substantially perpendicularly from the passage such that blood plasma flowing from the exit gates such that the blood plasma is distributed generally uniformly at the target treatment site thereby preventing pressure spikes associated with a single stream of blood plasma from a conventional needle.

The cross-sectional area of the exit gate may be smaller at the passage compared to the outlet of the exit gate. This is beneficial as the increasing cross-sectional area of the at least one exit gate helps to reduce the velocity of fluids, for example blood plasma, exiting the exit gates. This in turn helps to promote a uniform distribution of blood plasma at the target treatment site and the reduced fluid velocity and pressure reduces the chance of damaging the maxillary sinus lining membrane.

In one embodiment the body may comprise a distal shoulder extending around an external surface of the body. The distal shoulder beneficially may contact the maxillary bone, in use, and form a seal between the nozzle and the bone. The seal beneficially ensures that blood plasma exiting the outlet of the nozzle is distributed within the patient's sinus cavity and does not leak out of the target treatment site and into the patient's mouth. Furthermore, the seal encourages a gradual building of pressure at the target treatment site which may cause the maxillary sinus lining membrane (Schneiderian Membrane) to lift off the maxillary bone such that blood plasma may be distributed within the cavity created by the lifting of the maxillary sinus lining membrane from the maxillary bone.

In an embodiment the distal end of the body may extend distally from the distal shoulder. Furthermore, the distal end of the body may taper in a distal direction from the distal shoulder towards the distal tip along the longitudinal axis. This is beneficial as the distally tapering distal end makes it easier to locate the distal tip within the hole in the maxillary bone. This will also aid in creating the seal, prior to injecting the platelet rich blood plasma, should there be some discrepancies created in the access hole diameter, due to inherent anatomical features or the drilling process. The distal end of the body may be frustoconical such that the distal tip is generally planar.

In another embodiment the body may comprise a proximal shoulder extending around the external surface of the body. The proximal shoulder beneficially provides an abutment surface for a flexible tube that may be pressed over the proximal end of the nozzle to secure the nozzle to a syringe. The proximal end of the body may extend proximally from the proximal shoulder and the proximal end of the body may taper in a proximal direction from the proximal shoulder towards the proximal end along the longitudinal axis. Beneficially, this taper aids in the creation of frictional retention and seal of the flexible tube to the nozzle. As such, the proximal end of the body may be frustoconical such that the proximal tip is generally planar or flat.

In one embodiment the inlet may be positioned on the proximal tip and the inlet may be concentrically aligned with the longitudinal axis of the body. The inlet may be in fluid communication with the passage and the passage may convey fluids from the inlet to the outlet.

In an embodiment a central body portion may be defined between the distal shoulder and the proximal shoulder. The central body portion may taper in a proximal direction along the longitudinal axis from the distal shoulder towards the proximal shoulder. This is beneficial as the central body portion may provide a surface that a dental surgeon may grip. Furthermore, the proximally tapering central body portion allows the dental surgeon to push or urge the nozzle towards the maxillary bone to maintain a seal between the distal shoulder and the maxillary bone. Alternatively, the central body portion may have substantially parallel sides such that the cross-sectional area of the central body portion is substantially constant along the longitudinal axis between the proximal and distal shoulders. The outer surface of the central body portion may further comprise a textured surface so as to provide improved grip to the dental surgeon.

In one embodiment the nozzle may comprise four exit gates positioned circumferentially around the distal end of the body. Each exit gate may extend substantially perpendicularly from the passage to an external surface of the distal end of the nozzle. The at least one exit gate or all four exit gates may be generally square shape. In another embodiment the nozzle may comprise two, three or five exit gates.

As part of the invention there is further provided a syringe for delivering blood plasma to a target treatment site comprising a nozzle. Optionally, the nozzle is attached to, and in fluid communication with, the syringe via a flexible tube.

There is further provided an osteotome, the osteotome comprising: a body having a longitudinal axis; the body having a distal end, and the body comprising a distal shoulder extending around an external surface of the body; the distal end of the body extending distally from the distal shoulder, and wherein the distal end of the body tapers in a distal direction from the distal shoulder towards a distal tip along the longitudinal axis.

The taper and shoulder may beneficially allow a dentist to use the distal end of the osteotome to create an access opening having a maximum depth in a bone of a patient, particularly, but not limited to, the maxillary bone of a patient. The maximum depth of access opening that the osteotome can create is equal to the distance between the distal shoulder of the osteotome towards a distal tip of the osteotome along the longitudinal axis

Optionally, the distal end of the body of the osteotome is frustoconical such that the distal tip is generally planar. The distal tip of the osteotome being generally planar may provide the advantage that a planar tip is less likely to damage the Schneiderian Membrane, if the osteotome is used in a patient's maxillary bone.

There is also provided a kit for creating a hole in the maxillary bone of a patient, the kit comprising: a first osteotome and a second osteotome, the first osteotome having a first distance between the distal shoulder towards a distal tip along the longitudinal axis; the second osteotome having a second distance between the distal shoulder towards a distal tip along the longitudinal axis; the first and the second distances being non equal.

Optionally, the kit further comprises a third osteotome having a third distance between the distal shoulder towards a distal tip along the longitudinal axis; the third distance being non equal to either the first or the second distances.

When a kit of osteotomes is provided, the kit including osteotomes that have different dimensions, this allows a dentist/surgeon to use the kit of osteotomes to iteratively use osteotomes of increasing size/dimensions to gradually enlarge an access hole in the bone of a patient. By selecting an osteotome that has an appropriate distance between its distal shoulder and its distal tip, a dentist/surgeon has much greater control over the depth of access hole that they drill. If the kit of osteotomes are used in a patient's maxillary bone, this allows a dentist/surgeon to select an osteotome with dimensions that should not protrude too far through the maxillary bone, and are therefore much less likely to damage the Schneiderian Membrane of the patient.

There is also provided a kit for use in surgery, the kit comprising: an osteotome, and a nozzle for a syringe, and wherein: the length of the distal end of the body of the nozzle matches the length of the distal end of the body of the osteotome.

There is also provided a kit for use in surgery, the kit comprising: an osteotome, and a nozzle for a syringe, and wherein: the taper from the distal shoulder towards the distal tip of the body of the nozzle matches the taper from the distal shoulder towards the distal tip of the body of the osteotome.

There is also provided a kit for use in surgery, the kit comprising an osteotome, and/or a kit for use in surgery comprising an osteotome, and: and a nozzle for a syringe, the nozzle for a syringe comprising: a body having a longitudinal axis; the body having a distal end; and the body comprising a distal shoulder extending around an external surface of the body; the distal end of the body extending distally from the distal shoulder, and wherein the distal end of the body tapers in a distal direction from the distal shoulder towards a distal tip along the longitudinal axis and wherein: the length of the distal end of the body of the nozzle matches the length of the distal end of the body of either: the osteotome, or one of the osteotomes in the kit of osteotomes.

There is also provided a kit for use in surgery, the kit comprising an osteotome, and/or a kit for use in surgery comprising an osteotome, and: and a nozzle for a syringe, the nozzle for a syringe comprising: a body having a longitudinal axis; the body having a distal end, and the body comprising a distal shoulder extending around an external surface of the body; the distal end of the body extending distally from the distal shoulder, and wherein the distal end of the body tapers in a distal direction from the distal shoulder towards a distal tip along the longitudinal axis, and wherein: the taper from the distal shoulder towards the distal tip of the body of the nozzle matches the taper from the distal shoulder towards the distal tip of the body of either: the osteotome, or one of the osteotomes in the kit of osteotomes.

When the shape and dimensions of nozzle in a kit match the shape and dimensions of the largest osteotome used to drill an access hole into which the nozzle needs to be inserted, the match between the dimensions of the nozzle and the access hole allows a tight fit between the nozzle and the access hole, which allows increased continuous pressure to be delivered during delivery of the plasma and reduced leakage of plasma back into the mouth.

There is further provided a thumb tab for a plunging a plunger of a syringe, the thumb tab comprising: a base portion for attachment to the plunger; and a resiliently deformable spring element extending from the base portion; wherein the spring element is moveable from a relaxed position to a compressed position in response to a user pressing on the spring element to depress the plunger within the syringe, in use.

The thumb tab beneficially dampens changes in velocity of the plunger as it is plunged in the syringe as a result in variations in the pressure applied to the plunger by a user. When the spring element is in the compressed position variations in the pressure applied to the spring element by a user are dampened by the spring element which in turn promotes a smooth depression of the plunger within the syringe. This is particularly beneficial for applying fluids, for example blood plasma, to a target treatment site where pressure spikes in the fluid may damage the target treatment site.

Optionally, the spring element may be a deformable tab. For example, the deformable tab may be a plastics tab extending from the base portion of the thumb tab. The deformable tab may comprise a flat surface upon which a user may position their thumb to depress the plunger within the syringe.

Optionally, the deformable tab may be connected to a front edge, or distal edge, of the base portion. The spring element may extend generally upwardly, at least initially, away from the base portion. For example, the deformable tab may extend at an angle of about 45 degrees or more away from the front edge of the base portion. As such, the deformable tab may extend generally upwardly and in a proximal direction relative to the front edge of the base portion.

Optionally, the deformable tab may extend along an arcuate path from the base portion. For example, the arcuate path may extend in an upward and proximal direction relative to the front edge of the base portion. The deformable tab may be curved. Furthermore, a proximal end of the deformable tab may be generally flat to define a surface upon which the user may apply a force with their thumb to move the tab from the relaxed position to the compressed position in order to depress the plunger within the syringe.

The deformable tab may comprise an aperture. The aperture may be a slot extending through the deformable tab. This is beneficial as the dimensions of the aperture may be varied to control the stiffness of the deformable tab.

Optionally, the thumb tab may be integrally formed with the plunger. For example, the base portion of the thumb tab may be integrally formed with a plunger.

Optionally, the thumb tab may comprise an attachment formation on an underside of the base portion for securing the thumb tab to the plunger. As such, the thumb tab may be clipped on or secured to conventional plungers. This is beneficial as conventional plungers and syringes may be fitted with the thumb tab to promote smooth plunging of the plunger for applications where care should be taken to avoid pressure spikes in the fluid being expelled from the syringe.

The attachment formation may comprise an attachment flange coupled to the base portion. The attachment flange may be coupled to a front edge of the base portion. Furthermore, the attachment flange may extend in a proximal direction relative to the front edge to define a slot between the base portion and the attachment flange. The slot may be dimensioned so as to at least partially accommodate the top of a plunger within the slot. The slot may be dimensioned such that there is a push fit or press fit between the slot and the top of the plunger to retain the thumb attachment on the plunger.

Optionally, the attachment flange may comprise a notch on a proximal side of the attachment flange for at least partially accommodating the shaft of the plunger. A longitudinally extending recess may be positioned in a base of the notch for accommodating part of the shaft of the plunger. This is particularly beneficial for plungers having a shaft with a cross shaped profile.

Optionally, the spring element may comprise at least one grip formations for providing grip to a user of the thumb tab.

Optionally, the base portion may comprise an upwardly extending lip for inhibiting movement of the spring element. The lip may extend upwardly from a rear edge of the base portion. The lip beneficially inhibits movement of the spring element which in turn may improve the control of the plunger as a user applies a force to the spring element to depress the plunger.

There is further provided a plunger for a syringe wherein the plunger comprises a thumb tab.

There is further provided a syringe comprising a plunger.

There is further provided: a method of augmenting bone at a target treatment site on the patient's maxillary bone, the method comprising: inserting a nozzle into an access hole in the maxillary bone at the target treatment site; forming a seal between the nozzle and the maxillary bone; and distributing platelet rich blood plasma at the target treatment site through the access hole; wherein distributing the platelet rich blood plasma comprises lifting the patient's maxillary sinus lining off the maxillary bone to create a cavity and filling the cavity with the platelet rich blood plasma.

Optionally, lifting the patient's maxillary sinus may comprise applying a positive pressure to the sinus lining by injecting the platelet rich blood plasma at the target treatment site to cause the sinus lining to lift off the maxillary bone. Injecting the platelet rich blood plasma at the target treatment site may cause tenting of the maxillary sinus lining.

Optionally, the method may be preceded by drilling the access hole in the maxillary sinus. The dental surgeon may open or lift a flap in the gum to access the maxillary bone before drilling the access hole. The access hole beneficially allows the nozzle to be positioned so as to distribute blood plasma at the target treatment site.

There is further provided a method of drilling an access hole in the maxillary bone at the target treatment site, the method comprising:
using an osteotome comprising; a body having a longitudinal axis; the body having a distal end, and the body comprising a distal shoulder extending around an external surface of the body; the distal end of the body extending distally from the distal shoulder, and wherein the distal end of the body tapers in a distal direction from the distal shoulder towards a distal tip along the longitudinal axis,
to create an access hole with a depth equal to the distance between the distal shoulder towards the distal tip along the longitudinal axis of the osteotome, the osteotome being prevented from creating a deeper access hole due to abutment of the distal shoulder of the osteotome against the maxillary bone.

Optionally, the method of drilling an access hole in the maxillary bone at the target treatment site further comprises:
using a second osteotome comprising; a body having a longitudinal axis; the body having a distal end, and the body comprising a distal shoulder extending around an external surface of the body; the distal end of the body extending distally from the distal shoulder, and wherein the distal end of the body tapers in a distal direction from the distal shoulder towards a distal tip along the longitudinal axis,
to create an access hole with a depth equal to the distance between the distal shoulder towards the distal tip along the longitudinal axis of the second osteotome, the second osteotome being prevented from creating a deeper access hole due to abutment of the distal shoulder of the osteotome against the maxillary bone,
and, the distance between the distal shoulder towards the distal tip along the longitudinal axis of the second osteotome being greater than the distance between the distal shoulder towards the distal tip along the longitudinal axis of the first osteotome.

Optionally, the method of drilling an access hole in the maxillary bone at the target treatment site further comprises: repeating the step carried out by the second osteotome with one or more further osteotomes that have a distance between the distal shoulder towards the distal tip along the longitudinal axis of the osteotome being greater than the distance between the distal shoulder towards the distal tip along the longitudinal axis of the first or the second osteotome.

When the shape and dimensions of the nozzle inserted into an access hole drilled by an osteotome correspond to the shape of the largest osteotome used to drill the said access hole, the match between the dimensions of the nozzle sand the access hole allows a tight fit between the nozzle and the access hole, which allows increased continuous pressure to be delivered during delivery of the plasma and reduced leakage of plasma back into the mouth.

Optionally, distributing the platelet rich blood plasma may comprise ejecting the blood plasma from a plurality of exit gates in the nozzle. The exit gates may be spaced circumferentially around a distal end of the nozzle. Optionally, forming the seal may comprise pressing a distal shoulder of the nozzle against the maxillary bone. Beneficially, the seal between the nozzle and the maxillary bone allows a pressure to gradually build at the target treatment site as the platelet rich blood plasma is expelled from the nozzle. This in turn causes the maxillary sinus lining to lift off the maxillary bone.

There is also provided a method of augmenting bone at a target treatment site, the method comprising: inserting a nozzle into an access hole in a bone at the target treatment site; forming a seal between the nozzle and the bone; and distributing platelet rich blood plasma at the target treatment site through the access hole.

This method of augmenting bone may be used, for example, to augment bone at a target treatment site on a patient's skull. The nozzle may be inserted into an access hole in the patient's skull and platelet rich blood plasma may be distributed at the target treatment site using the nozzle. The seal formed between the bone and the nozzle beneficially ensures the platelet rich blood plasma is distributed at the target treatment site. The method may be used with the nozzle and or thumb tab.

There is also provided a method of expelling a fluid, such as platelet rich blood plasma from a syringe wherein the syringe comprises a plunger having a deformable thumb tab, the method comprising: applying a plunging force to a spring element on the thumb tab; moving the spring element from a relaxed position to a compressed position in response to applying the plunging force to the spring element; and plunging the plunger within the syringe to expel the fluid from the syringe whilst the spring element is in the compressed position.

Within the scope of this application it is expressly intended that the various aspects, embodiments, examples and alternatives set out in the preceding paragraphs, and/or in the following description and drawings, and in particular the individual features thereof, may be taken independently or in any combination.

### BRIEF DESCRIPTION OF THE DRAWINGS

The invention will now be described, by way of example only, with reference to the accompanying drawings, in which:
Figure 1 is a schematic sectional view of a target treatment site within a patient's maxillary sinus cavity;
Figure 2 is a side view of a syringe nozzle according to an embodiment of the invention;
Figure 3 is a perspective view of the distal end of the syringe nozzle of Figure 2;
Figure 4 is a perspective view of the proximal end of the syringe nozzle of Figure 2;
Figure 5 is a schematic sectional view of the syringe nozzle of Figure 2 positioned at the target treatment site of Figure 1;
Figure 6 is a perspective view of a thumb tab for a syringe;
Figure 7 is a side view of the thumb tab of Figure 6 in a relaxed configuration;
Figure 8 is a side view of the thumb tab of Figure 6 in a compressed configuration;
Figure 9 is a perspective view of the underside of the thumb attachment of Figure 6;
Figure 10 is a perspective view of a thumb tab for a;
Figure 11 is a perspective view of a syringe comprising the thumb tab of Figure 6 and the nozzle of Figure 2;
Figure 12a and 12b are side view of the thumb tab of Figure 6 and Figure 10 respectively integral with a plunger;
Figure 13 is a perspective view of the syringe of Figure 10 being held and operated by a user;
Figure 14 is a perspective view of an osteotome;
Figure 15 is a series of side views of six osteotomes;

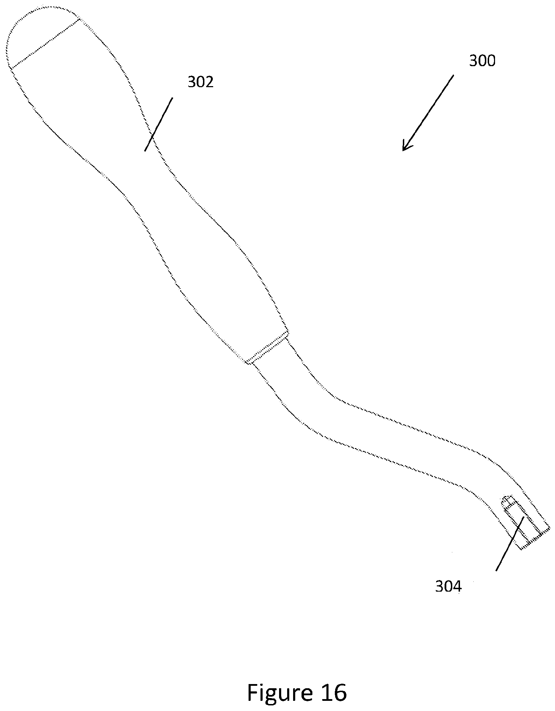

Figure 16 is a handle for use with an osteotome; and
Figure 17 is a flow chart outlining a method of delivering platelet-rich blood plasma to a target treatment site;

### DETAILED DESCRIPTION

In general terms there is provided a nozzle for a syringe and to a thumb tab for a plunger for use with a syringe for delivering blood plasma to a target treatment site within a sinus cavity of a patient's mouth, and an osteotome for drilling an access hole to a sinus cavity of a patient's mouth, suitable for receiving a nozzle of a syringe. The nozzle may be fitted to a syringe such that fluid, for example blood plasma, can be delivered to a target treatment site within the patient's mouth. The nozzle comprises a body having an inlet for receiving blood plasma from the syringe and an outlet for delivering the blood plasma to the target treatment site within the sinus cavity of the patient. The outlet of the nozzle comprises an outlet aperture located on a distal tip of the nozzle aligned with a longitudinal axis of the nozzle and a plurality of exit gates positioned circumferentially around the distal end of the nozzle.

The nozzle beneficially allows blood plasma to be dispersed evenly at the target treatment site through the outlet aperture and exit gates. Dispersing the blood plasma evenly at the target treatment site is beneficial as dispersed flow from the outlet reduces pressure spikes or pressure points on the sinus lining membrane which may cause the sinus lining membrane to burst or rupture.

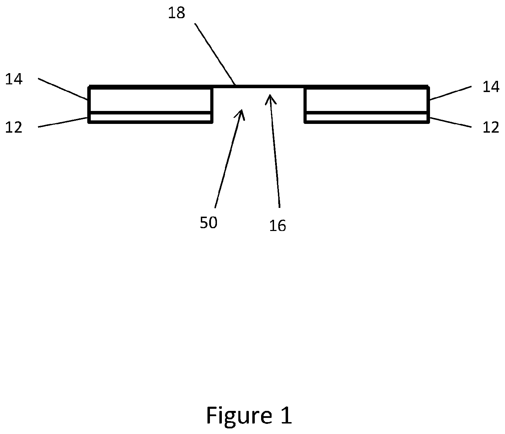

Reference will firstly be made to Figure 1 which shows a schematic sectional view of a patient's gum 12 and maxillary bone 14 at a target treatment site 16 within a patient's mouth. The target treatment site 16 may be a site where a dental implant is to be fitted and augmentation of the maxillary bone 14 is required prior to fitting the implant. For example, the target treatment site 16 may be a region of the maxillary bone 14 where augmentation of the bone 14 is required in order to grow sufficient bone to allow a dental implant to be fitted at the target treatment site 16. The maxillary sinus membrane 18 is positioned above the maxillary bone 14 and extends across the target treatment site 16. As shown in Figure 1 the maxillary sinus lining membrane 18 extends over the maxillary bone 14.

In the example shown the patient does not have any teeth adjacent the target treatment site 16 but the skilled reader will understand that in some instances the patient may have teeth on one or both sides of the target treatment site 16. The skilled reader will understand that the target treatment site may also be remote from the proposed dental implant site. In this instance the access hole is not sealed with the placement of a dental implant but a non-resorbable membrane is placed over the access hole (osteotomy) during the healing phase. This non-resorbable membrane will be removed once healing is complete, usually 3 - 6 months after the initial treatment.

To access the target treatment site 16 a dentist may initially anaesthetise the patient and create a flap (not shown) within the patient's gum 12 to expose and access the maxillary bone14. A hole may subsequently be drilled in the maxillary bone 14 such that platelet rich blood plasma may be delivered to the target treatment site 16 via the hole in the bone 14.

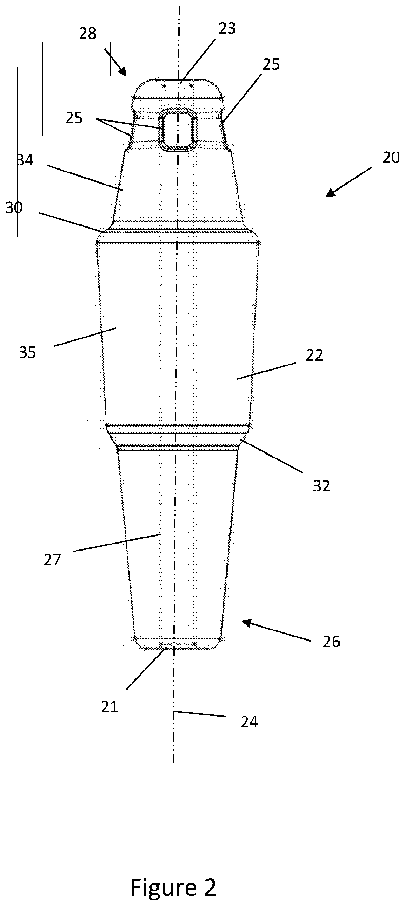

Turning now to Figure 2 a nozzle 20 for a syringe is shown. The syringe nozzle 20 is configured to distribute platelet rich blood plasma at the target treatment site 16 within the patient's mouth. The syringe nozzle 20 comprises a body 22 having a longitudinally extending axis 24. The longitudinal axis 24 extends between a proximal end 26 of the body 22 and a distal end 28 of the body 22. A passage 27 for conveying blood plasma from the syringe to the target treatment site 16 extends through the body 22 along the longitudinal axis 24. As such, the passage 27 extends between and fluidly connects the proximal end 26 to the distal end 28 of the nozzle 20 through the body 22.

The proximal end 26 of the nozzle 20 comprises an inlet 21 for receiving blood plasma from a syringe (not shown in Figure 2). Furthermore, the outlet of the nozzle 20 is located at the distal end 28 of the nozzle 20 and comprises an outlet aperture 23 and radially extending exit gates 25. The outlet aperture 23 and exit gates 25 are positioned at the distal end 28 of the nozzle 20 for delivering and distributing blood plasma at the target site 16 of the patient. The passage 27 extends from the inlet 21 to the outlet aperture 23 such that the inlet aperture 21, outlet aperture 23 and exit gates 25 are fluidly connected via the passage 27.

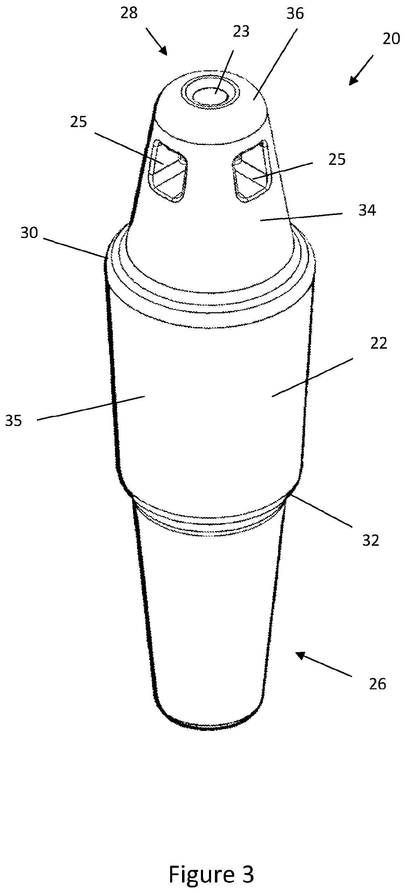

As shown in Figure 3 the body 22 of the nozzle 20 comprises a central body portion 35. The distal end 28 and the proximal end 26 extend in distal and proximal directions respectively from the central body portion 35. As shown in Figure 3 the distal end 28 of the nozzle 20 is frustoconical in shape with a generally planar distal tip 36. The frustoconical distal end 28 extends and tapers distally from a distal shoulder 30 of the central body portion 35. The side wall 34 of the distal end 28 tapers in a distal direction along the longitudinal axis 24 from the shoulder 30 towards the distal tip 36.

Four exit gates 25 are arranged circumferentially around the distal end 28 of the nozzle 20 such that the exit gates 25 extend radially from the passage 27 and longitudinal axis 24, through the side wall 34 of the distal end 28. Each exit gate 25 is generally square in shape. This is beneficial as the square shape of the exit gates 25 maximises the cross-sectional area of each gate 25 which in turn helps to promote a uniform distribution and smooth flow from each gate 25 thereby reducing the chance of a pressure spike in the fluid rupturing or tearing the sinus lining membrane.

Furthermore, the distally tapering side wall 34 and the position of the exit gates 25 on the side wall 34 allow the nozzle 20 to be used in situations where the bone 14 is thicker than the length of the distal end 28. In this scenario the tapering distal end 28 provides clearance between the exit gates 25 and the bone 14 such that blood plasma may still be distributed at the target treatment site 16.

Furthermore, each exit gate 25 is tapered such that the cross-sectional area of each exit gate 25 increases from the passage 27 towards the outer surface of the side wall 34. This is beneficial as the increasing cross-sectional area of the exit gates 25 reduces the velocity of fluid being expelled from the exit gate 25 which in turn reduces the resultant pressure on the maxillary sinus 18 resulting from blood plasma exiting the exit gates 25.

The outlet aperture 23 is positioned on, and extends through, the distal tip 36 of the nozzle 20 such that the outlet aperture 23 is concentrically aligned with the longitudinal axis 24. The passage 27 extends through the distal end 28 of the nozzle 20 such that the outlet aperture 23 and exit gates 25 are in fluid communication with the passage 27. The outlet aperture 23 may be a circular aperture having a diameter of between about 0.5mm and 2mm. The relatively small diameter outlet aperture 23 beneficially reduces the flow of material from the outlet aperture 23 thereby reducing the chance of the maxillary sinus lining 18 being ruptured by pressure spikes in blood plasma being expelled from the outlet aperture 23.

The central body portion 35 comprises a distal shoulder 30 and a proximal shoulder 32. The distal and proximal shoulders 30, 32 extend around the distal and proximal ends 26, 28 of the central body portion 35. This is beneficial as the distal shoulder 30 may engage and rest upon the patient's gum 12 or maxillary bone 14 when the nozzle 20 is being used to deliver blood plasma to the target treatment site 16. As such, the distal shoulder 30 may form a seal between the nozzle 20 and the gum 12 or maxillary bone 14 as is discussed in further detail below. Furthermore, the proximal shoulder 32 may act as a stop for a flexible tube or hose connecting the nozzle 20 to a syringe. For example, a hose may be pushed over the proximal end 26 such that it abuts and seals against the proximal shoulder 32.

The central body portion 35 tapers in a proximal direction along the longitudinal axis 27 from the distal shoulder 30 towards the proximal shoulder 32. As such, the diameter of the central body portion 35 is greater at the distal shoulder 30 compared to the proximal shoulder 32. This is beneficial as the proximally tapering central body portion 35 provides a surface that a dentist may grip when using the nozzle 20. The central body portion 35 may be textured to improve grip. Furthermore, the tapering central body portion 35 prevents the dentist's fingers from sliding down the nozzle 20, in use, in a distal direction towards the target treatment site 16. This is beneficial as the dentist may push or urge the nozzle 20 towards the hole in the maxillary bone 14 to ensure a seal is created between the distal shoulder 32 and the maxillary bone 14 or gum 12.

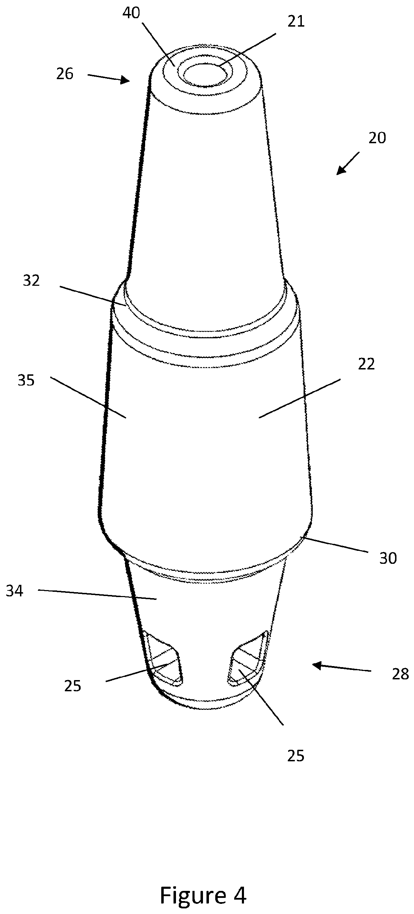

As best viewed in Figure 4, the proximal end 26 of the nozzle 20 is frustoconical shaped and comprises a generally planar proximal tip 40. The proximal end 26 of the nozzle 20 extends and tapers in a proximal direction along the longitudinal axis 24 from the proximal shoulder 32 of the central body portion 35 to a proximal tip 40. The passage 27 extends along the longitudinal axis 24 through the proximal end 26 and terminates at the inlet 21 on the proximal tip 40. The inlet 21 is positioned on the proximal tip 40 and is aligned concentrically with the central longitudinal axis 24 and thus also the outlet aperture 23.

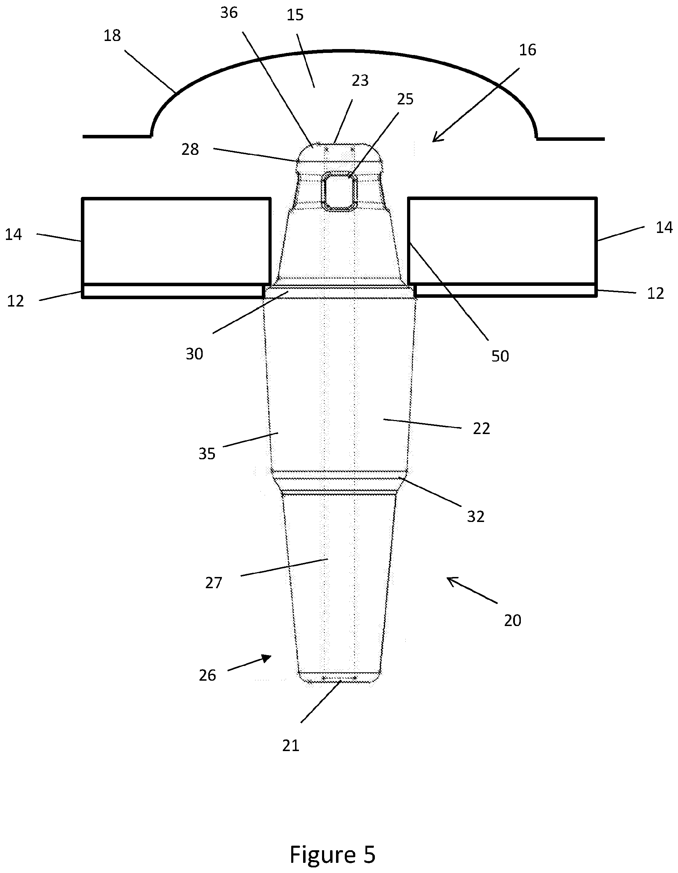

Turning now to Figure 5 the nozzle 20 is shown schematically at the target treatment site 16 within the patient's mouth. To position the nozzle 20 at the target treatment site 16 the dentist may open a flap (not shown) in the patient's gum 12 at the target treatment site 16 where augmentation of the maxillary bone 14 is required. A small hole 50, for example between about 2mm - 5mm diameter is drilled in the maxillary bone 14 to gain access to the sinus membrane 18 and the distal end 28 of the nozzle 20 may be at least partially received within the hole 50.

When the nozzle 20 is positioned at the target treatment site 16 the distal shoulder 30 abuts the gum 12 and/or maxillary bone 14 to form a seal between the nozzle 20 and the maxillary bone 14. The distal end 28 is positioned at the target treatment site 16 such that the distal tip 36 protrudes through the hole 50 in the maxillary bone 14 and the distal end 28 is received within the hole 50.

The platelet rich blood plasma is expelled through the outlet aperture 23 and exit gates 25 such that the blood plasma is distributed evenly at the target treatment site 16. As the platelet rich blood plasma is expelled from the outlet of the nozzle 20 the build-up of pressure from the blood plasma causes the maxillary sinus membrane 18 to lift off the maxillary bone 14 as shown in Figure 5 to create a cavity 15. The seal between the distal shoulder 30 and the maxillary bone 14 causes an increase in pressure within the cavity 15 as the blood plasma is injected to the target treatment site 16. The increase in pressure causes the maxillary sinus membrane 18 to lift off the bone 14 to create the cavity 15 which is subsequently filled by platelet rich blood plasma. The nozzle 20 is then removed from the patient's mouth leaving the blood plasma in the target treatment site 16. The access hole is then sealed with a dental implant or a non-resorbable membrane and the gum sutured back in place, over the dental implant or non-resorbable membrane.

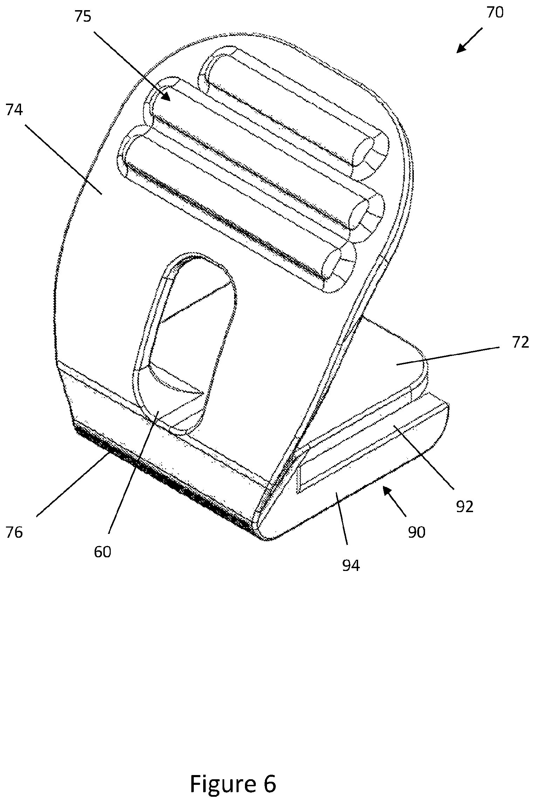

Turning now to Figure 6 a thumb tab 70 for plunging a plunger of a syringe is shown. In general terms the thumb tab 70 comprises a base portion 72 for attachment to a plunger of a syringe and a deformable tab or spring element 74 extending from the base portion 72 for depressing the plunger of the syringe. The base portion 72 may be integral with a plunger or the base portion 72 may comprise an attachment formation 90 for securing the thumb tab 70 to a plunger of a conventional syringe.

The spring element 74 of the thumb tab 70 is resiliently deformable such that when a user of the syringe depresses the plunger by pressing on the spring element 74 of the thumb tab 70 the spring element 74 is compressed such that it bends or flexes. The spring element 74 may be moved from a relaxed position to a compressed or loaded position, in response to a user pressing on the thumb tab 70 to depress the plunger. The spring element further comprises a series of gripping formations 75 in the form of laterally extending ridges on an upper surface of the spring element 74. The gripping formations 75 provide a surface upon which a user may apply a plunging force with their thumb to the spring element 74.

When the spring element 74 is in the compressed position the spring element 74 may act to dampen fluctuations in the velocity of the depression of the plunger caused by variations in the plunging force applied to the plunger by a user. This in turn promotes a smoother depression of the plunger and thus reduces pressure spikes in the fluid expelled from the syringe. This is particularly beneficial for applying platelet rich blood plasma to a target treatment site 16 within a sinus cavity 15 as spikes in the pressure of the fluid may rupture the delicate maxillary sinus membrane 18.

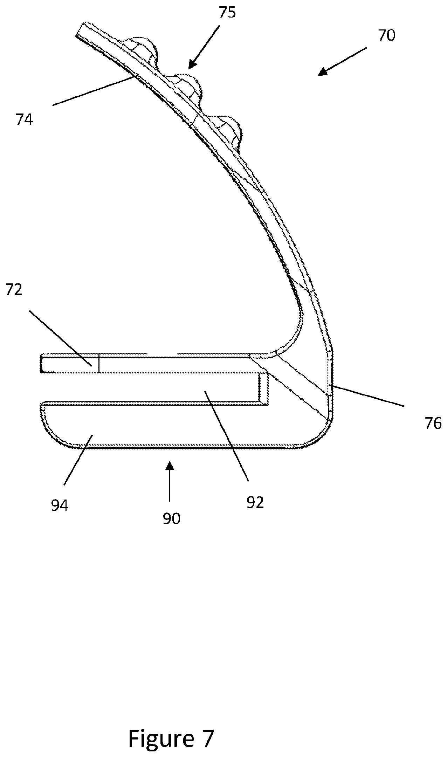

Figure 7 shows a side view of the thumb tab 70 in the relaxed position. As shown in Figure 7 the side profile of the spring element 74 is generally arcuate or curved. The spring element 74 extends generally upwardly away from a front edge 76 of the base portion 72 initially before curving in a proximal direction relative to the front edge 76 of the thumb tab 70.

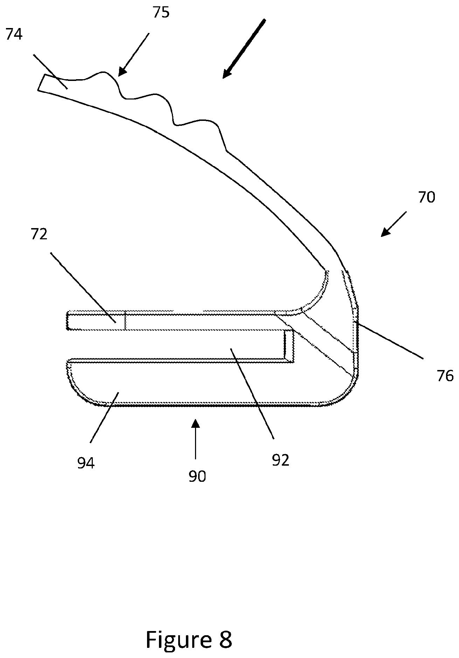

Figure 8 shows a side view of the thumb tab 70 in the compressed position. In Figure 8 the spring element 74 of the thumb tab 70 has been depressed towards the base portion 72 as would be the case when a user of the syringe applied a load on the thumb tab 70 to depress a plunger. When in the compressed position the force applied to the spring element 74 is balanced by the reactant force from the spring element 74 and the force associated with depressing the plunger. Any variations in the pressure applied to the plunger by the user may be dampened by movements in the spring element 74 such that the force applied to the plunger is substantially constant thereby reducing pressure spikes in the blood plasma exiting the syringe.

As best viewed in Figure 6, an aperture 60 is positioned within the spring element 74. The aperture 60 may be a slot extending upwardly from the front edge 76 of the base portion 72 through the spring element 74. The size of the aperture 60, in particular the width of the aperture 60, may be varied to alter the stiffness of the spring element 74. For example, if a relatively stiff spring element 74 is required the aperture may be relatively narrow, for example 1mm - 3mm wide or the aperture 60 may be removed entirely. Where a more flexible spring element 74 is required the width and length of the aperture 60 may be increased. For example, the width of the aperture 60 may be 4mm or greater.

The aperture 60 beneficially allows the thumb tab 70 to be tuned to be used with syringes of differing volumes. For example, a syringe with a relatively large volume may require a stiffer spring element 74 than a corresponding syringe having a smaller volume.

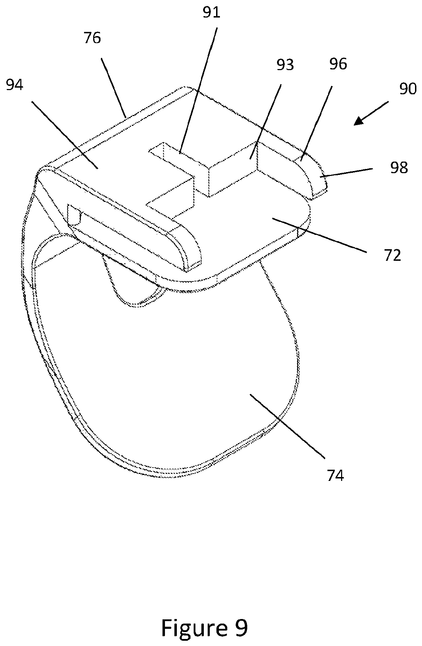

Turning now to Figure 9 an underside perspective view of the thumb tab 70 is shown. The base portion 72 comprises an attachment formation 90 for securing the thumb tab 70 to a plunger of a syringe. The attachment formation 90 is configured to allow the thumb tab 70 to be clipped to the plunger of a conventional syringe to dampen pressure spikes in the fluid being expelled from the syringe.

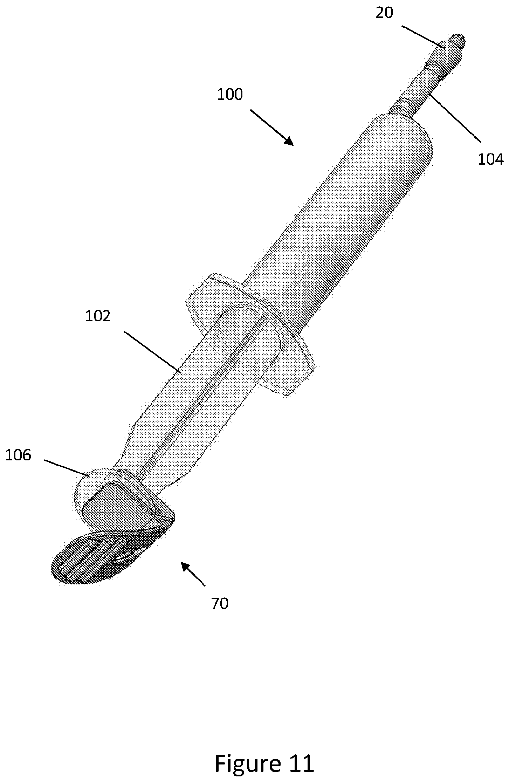

As shown in Figure 9 the attachment formation 90 may comprise an attachment flange 94 secured to the base portion 72 at the front edge 76 of the base portion 72. The attachment flange 94 extends substantially parallel to the base portion 72 in a proximal direction from the front edge 76. A slot 92 is defined between the underside of the base portion 72 and an upper side of the attachment flange 94. As such the slot 92 extends generally parallel to a plane of the base portion 72. The slot 92 is configured to at least partially receive the top of a plunger as shown in Figure 11. The thumb attachment 70 may be secured to the plunger by sliding the attachment flange 94 over the top of a plunger such that the top of the plunger is at least partially received within the slot 92.

The attachment flange 94 comprises a notch 96 on the proximal side 98 of the flange 94 for accommodating the shaft of the plunger. The shaft of the plunger typically comprises a cross-shaped profile and the notch 96 is dimensioned to at least partially the cross-shaped shaft of the plunger. The notch 96 further comprises a recess 91 in the base 93 of the notch 96. The recess 91 is a longitudinally extending recess or slot in the base 93 of the notch 96 for at least partially receiving one of the arms of the cross-shaped shaft of the plunger.

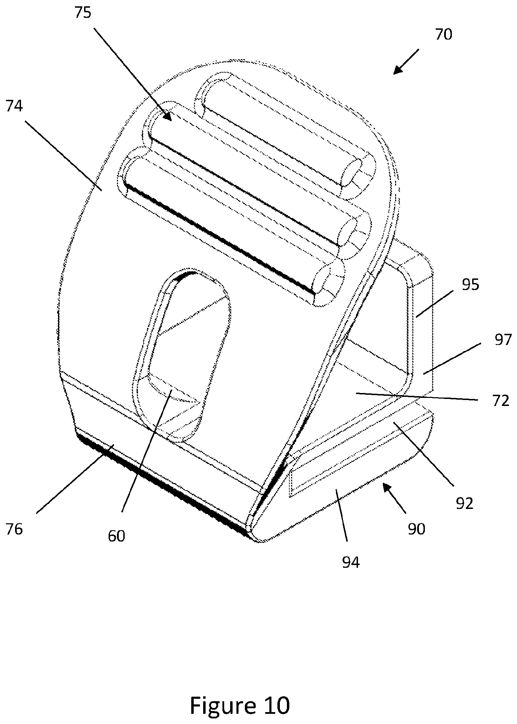

Figure 10 shows the thumb tab 70. As shown in Figure 10 the thumb tab 70 comprises an upwardly extending lip 95. The lip 95 serves as an abutment or stop to limit the movement of the spring element 74. Inhibiting the compression of the spring element 74 is beneficial as it improves control when plunging the plunger. The lip 95 is connected to and extends upwardly from a rear edge 97 the base portion 72.

When the spring element 74 is in the compressed position the spring element 74 typically does not contact the lip 95. However, if there was, for example, a blockage that prevented fluid exiting the nozzle 20 the spring element may be further deformed and contact the lip 95. Upon contact with the lip 95 the dental surgeon using the thumb tab 70 may realise that there is a blockage and stop applying pressure to the spring element 74. This beneficially prevents any pressure spikes in the fluid being distributed by the nozzle 20.

Turning now to Figure 11 there is shown a syringe 100 and plunger 102. The distal tip of the syringe 100 comprises the syringe nozzle 20 and the plunger 102 comprises the thumb tab 70.

As shown in Figure 11, the nozzle 20 is connected to the distal tip of the syringe 100 by a flexible tube 104. The flexible tube 104 may be made from a flexible rubber material, plastics material or the like. The flexible tube 104 beneficially allows the position of the nozzle 20 relative to the syringe 100 to be controlled by a dentist. For example, when the nozzle 20 is positioned at a target treatment site 16 the dentist may position the nozzle 20 such that it extends generally perpendicularly relative to the syringe 100 to ensure that the nozzle 20 is correctly positioned and sealed to the maxillary bone 14 at the target treatment site 16.

The thumb attachment 70 may be clipped to the circular top 106 of the plunger 104 via the attachment formation 90. As such, a user of the syringe 100 may press on the thumb tab 70 to depress the plunger 102 and expel platelet rich blood plasma from the nozzle 20 to the target treatment site.

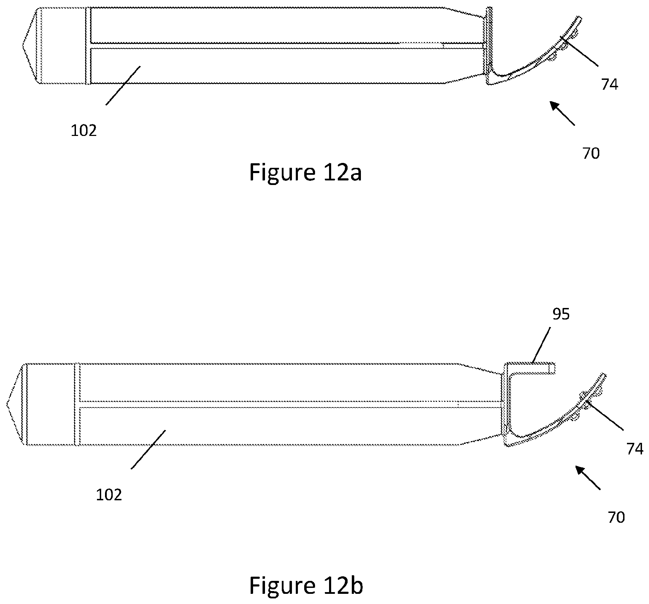

Whilst the above shows the thumb tab 70 as a component that may be clipped to the top 106 of the plunger 102, the thumb tab 70 may instead be an integral part of the top 106 of the plunger 102. Figures 12a and 12b show side views of plungers 102 where the thumb tab 70 is integral with or integrally formed with the plunger 102.

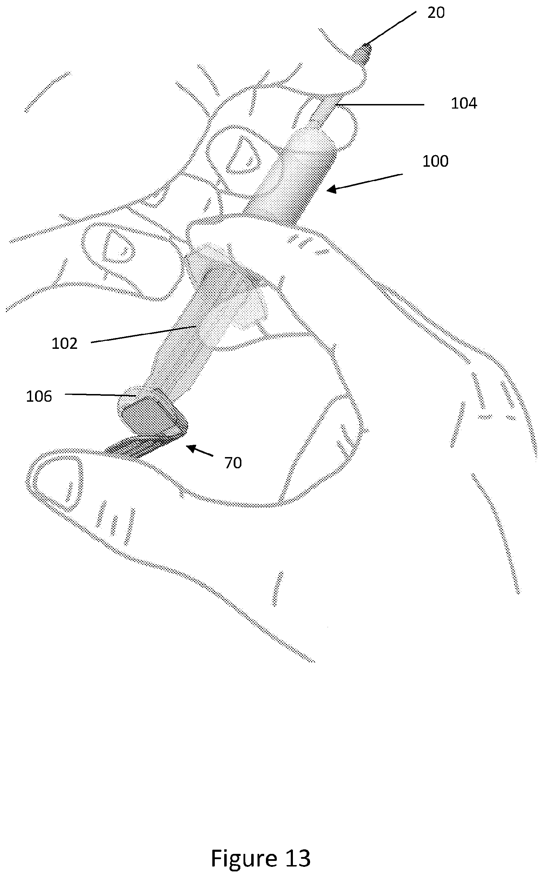

Turning now to Figure 13, the syringe 100 of Figure 10 is shown in the hands of a user, for example, in the hands of a dental surgeon. As shown in Figure 13, the user's right hand 110 may be used to depress the plunger 102 by placing the user's right-hand thumb on the thumb tab 70. The user may then apply a force to the thumb tab which will compress the thumb tab 70 to the compressed configuration and also depress the plunger 102 within the syringe 100. The user may continue to apply the downward pressure on the thumb tab 70 to expel the fluid within the syringe from the nozzle 20. Any fluctuations in the pressure applied to the thumb tab 70 by the user will be dampened by the spring element 74 thereby promoting a constant depression velocity of the plunger 102 and thus pressure spikes in the fluid expelled from the nozzle 20 will be minimised.

Furthermore, as shown in Figure 13 the user may use their other hand, in this case their left hand, to control the position of the nozzle 20 relative to the syringe 100. The user may grip the central body portion 35 of the nozzle 20 to grip the nozzle 20 and control the position of the nozzle 20 by manipulating the flexible tube 104. The skilled reader will understand that the user may alternatively use their left hand to depress the plunger 102 and to use their right hand 110 to hold and control the position of the nozzle 20.

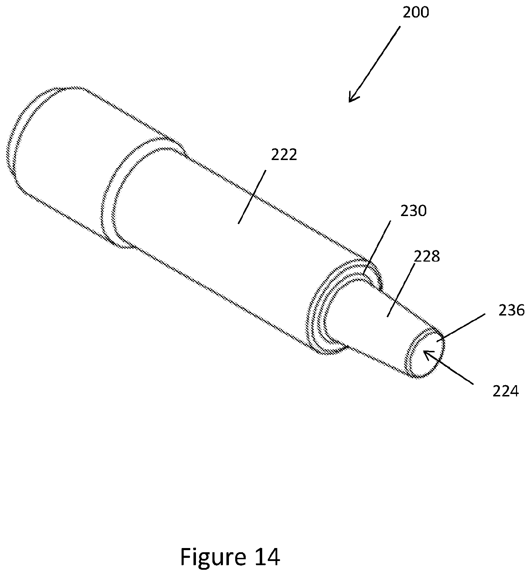

Figure 14 shows an osteotome 220. Osteotome 220 is configured to drill a hole 50 enabling a nozzle to access a treatment site 16. Osteotome 220 comprises a body 222 having a longitudinal axis 224; the body having a distal end 228, and the body 222 comprising a distal shoulder 230 extending around an external surface of the body; the distal end 228 of the body extending distally from the distal shoulder 230, and wherein the distal end 228 of the body tapers in a distal direction from the distal shoulder 230 towards a distal tip 236 along the longitudinal axis.

Distal end 228 of the body 222 of the osteotome 220 is frustoconical such that the distal tip 236 is generally planar. The distal tip of the osteotome being generally planar may provide the advantage that a planar tip is less likely to damage the Schneiderian Membrane, if the osteotome is used to drill through (or at least partially through) a patient's maxillary bone.

When the osteotome 220 has drilled a hole 50 as far as possible towards the target treatment site 16 the distal shoulder 230 of the osteotome 220 will abut the gum 12 and/or maxillary bone 14 to prevent the distal tip 236 of the osteotome 220 drilling any further through the maxillary bone 14. Each osteotome therefore has a maximum drilling depth that it can reach. In order to increase the depth of a hole 50 in the maxillary bone of a patient, it is preferable to then use another osteotome 220a also having a body 222a having a longitudinal axis 224a; the body having a distal end 228a, and the body 222a comprising a distal shoulder 230a extending around an external surface of the body; the distal end 228a of the body extending distally from the distal shoulder 230a, and wherein the distal end 228a of the body tapers in a distal direction from the distal shoulder 230a towards a distal tip 236a along the longitudinal axis.

In osteotome 220a, the distance between distal shoulder 230a and distal tip 236a along the longitudinal axis 224a is longer than the distance between distal shoulder 230 and distal tip 236 along the longitudinal axis 224 of first osteotome 220. This allows osteotome 220a to drill deeper into the maxillary bone of a patient than osteotome 220 can, before the shoulder 230a abuts the maxillary bone of the patient and prevents osteotome 220a from drilling any deeper.

If it is necessary to drill the hole 50 in the maxillary bone deeper still in order to reach the treatment site 16, it is preferable to use a further osteotome 220b. Osteotome 220b having a distal shoulder 230b and distal tip 236b, and the distance between distal shoulder 230b and distal tip 236b being greater than the distances between the distal shoulder 230, 230a and distal tip 236, 236a in osteotome 220 or in 220a. This therefore allows a surgeon to drill deeper into the maxillary bone of a patient than osteotome 220 or 220a can, before the shoulder 230b abuts the maxillary bone of the patient and prevents osteotome 220b from drilling any deeper.

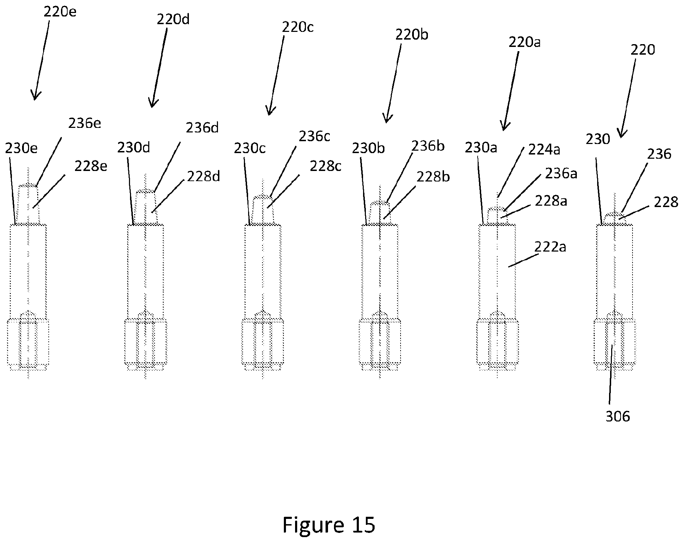

Providing a kit of osteotomes with different distances between their respective distal shoulders and distal tips allow a dentist/surgeon to select an osteotome that will only slightly increase the depth of the current hole 50. Providing a selection of osteotomes allows the surgeon to carefully, and very incrementally, increase the depth of hole 50 to gain access to treatment site 16, while keeping the odds low that the osteotome will protrude too far through the bone, meaning that the odds of damage to the Schneiderian Membrane of the patient decrease. Fig. 15 shows a kit of osteotomes 220, 220a, 220b, 220c, 220d, and 220e, having respective distal ends 228, 228a, 228b, 228c, 228d, and 228e, their respective distal ends having respective distal shoulders 230, 230a, 230b, 230c, 230d, and 230e, and respective distal tips 236, 236a, 236b, 236c, 236d, and 236e. As shown in Fig. 15, the distances between the respective distal shoulders 230, 230a, 230b, 230c, 230d, and 230e, and respective distal tips 236, 236a, 236b, 236c, 236d, and 236e of osteotomes 220, 220a, 220b, 220c, 220d, and 220e vary, enabling a surgeon to choose the most appropriate osteotome for the stage of hole drilling that they are at.

Any of the osteotomes can be used with a handle such as osteotome handle 300. osteotome handle 300 has a grip 302 for a user to grip, and a connector 304 to which an osteotome 220, 220a, 220b, 220c, 220d, or 220e may be attached. Any osteotome to be used with handle 300 may be provided with a connector 306 configured to connect to connector 304.

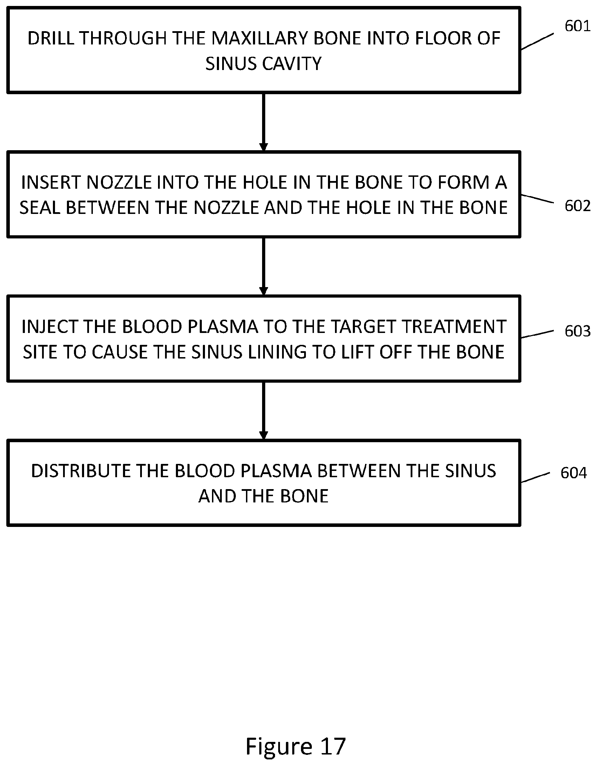

Figure 17 outlines a method of distributing platelet enriched blood plasma at a target treatment site 16 within a patient's mouth using the nozzle 20. In Step 601 the dental surgeon drills through the maxillary bone 14 into the floor of the maxillary sinus cavity, using an osteotome. Drilling through the maxillary bone 14 exposes the maxillary sinus 18 and provides access to the sinus membrane. The hole 50 drilled through the maxillary bone 14 may be between about 2mm and 5mm in diameter. For example, a dentist may use a 3mm diameter drill to create the hole 50 in the maxillary bone 14. Prior to Step 601 the dentist may apply local anaesthetic to the target treatment area 16 and open a gum flap in the patient's gum 12 to provide access to the maxillary bone 14 where the hole 50 is to be drilled.

In Step 602 the distal end 28 of the nozzle 20 is inserted into the hole drilled into the maxillary bone 14. The distal end 28 of the nozzle 20 is advanced into the hole until the distal shoulder 30 of the nozzle 20 abuts the maxillary bone 14 or gum 12 around the perimeter of the hole 50. The distal shoulder 30 forms a seal between the nozzle 20 and the maxillary bone 14 thereby preventing any blood plasma leaking out from the target treatment site 16 when the blood plasma is being distributed by the nozzle 20 at the target treatment site 16. Furthermore, the distal shoulder 30 may be used to control or inhibit the depth of penetration of the distal end 28 of the nozzle 20 into the sinus cavity 15 thereby stopping the dentist inadvertently tearing the sinus lining 18 with the distal end 28 of the nozzle 20.

It will be appreciated that using a nozzle having a distal end that corresponds in shape/size to the largest (final) osteotome used to drill the hole 50 will result in a close fit between the nozzle and the hole 50. Having a tight fit between the nozzle and the hole drilled by the osteotome allows increased continuous pressure to be delivered during delivery of the plasma and reduced leakage of plasma back into the mouth in step 603.

In Step 603 the platelet rich blood plasma is injected or distributed at the target treatment site 16 via the nozzle 20. When the blood plasma is initially injected a pressure builds within the sinus cavity 15 at the target treatment site 16 as a result of the blood plasma being injected and also the seal formed by the distal shoulder 30. As the pressure at the target site builds the maxillary sinus lining or membrane 18 lifts off the maxillary bone 14 as shown in Figure 5 to create a cavity 15. As such, tenting or lifting of the maxillary sinus 18 lining occurs at the target treatment site 16 and platelet rich blood plasma fills the cavity 15 created between the maxillary bone 14 and the maxillary sinus 18.

In Step 604 blood plasma is continued to be distributed at the target treatment site 16 such that the sinus cavity 15 defined between the maxillary bone 14 and the sinus lining 18 that has been lifted off the maxillary bone 14 is filled with the platelet rich blood plasma. Distributing the blood plasma at the target treatment site 16 may comprise expelling the blood plasma in a direction aligned with the longitudinal axis 24 and a further direction that is generally perpendicular to the longitudinal axis 24. For example, the blood plasma may be expelled from the outlet aperture 23 and one or more of the exit gates 25.

When the blood plasma has been distributed at the target treatment site 16 the nozzle 20 may be removed from the hole 50 in the maxillary bone 14 and the flap in the gum 12 may be closed. The platelet rich blood plasma at the target treatment site 16 will promote growth and augmentation of the maxillary bone 14 such that a dental implant may be fitted at the target treatment site 16.

A kit including a range of sizes of osteotomes and nozzles enables a surgeon to test whether the treatment site 16 has been reached in a less invasive way than previous equipment allowed. One further advantage of using a set of osteotomes with varying distal end sizes/shapes and a set of nozzles with correspondingly varying distal end sizes/shapes is that the surgeon can, non-invasively, test whether they have successfully reached the treatment area 16 by removing an osteotome, inserting the correspondingly sized/shaped nozzle (attached to a syringe and filled with blood plasma) and gently

Applying pressure to the plunger of the syringe. If the plunger has not started to depress within 10-15 seconds it is unlikely that the hole 50 has fractured up through the floor of the sinus yet. Continuing to attempt to force the plasma through the nozzle could result in perforation of the sinus membrane and failure of the technique (on this attempt). To remedy this the next of osteotome is chosen, the hole 50 is extended, then the corresponding nozzle is used to reattempt to deliver plasma to the site 16. These steps are repeated, until it is possible to deliver the plasma gel into the sinus in a controlled manner.

It will be appreciated that various changes and modifications can be made to the present invention without departing from the scope of the present invention.

## Claims

1. A nozzle (20) for a syringe (100) for distributing blood plasma at a target treatment site (16) within a patient's mouth, the nozzle (20) comprising:
a body (22) having a passage (27) extending through the body (22) along a longitudinal axis (24) of the body (22), the passage (27) extending from a proximal end (26) to a distal end (28) of the body (22);
an inlet (21) to the passage (27) at the proximal end (26) for receiving blood plasma from the syringe (100);
an outlet from the passage (27) at the distal end (28) for distributing blood plasma at the target treatment site (16), wherein the outlet comprises an outlet aperture (23) located on a distal tip (36) of the nozzle (20); and
at least one exit gate (25) extending from the passage (27) through the distal end (28) of the body (22), **characterized in that** the at least one exit gate (25) tapers outwardly from the passage (27).

2. A nozzle (20) as claimed in Claim 1, wherein the body (22) comprises a distal shoulder (30) extending around an external surface of the body (22).

3. A nozzle (20) as claimed in Claim 2, wherein the distal end (28) of the body (22) extends distally from the distal shoulder (30) and wherein the distal end (28) of the body (22) tapers in a distal direction from the distal shoulder (30) towards the distal tip (36) along the longitudinal axis (24).

4. A nozzle (20) as claimed in Claim 3, wherein the distal end (28) of the body (22) is frustoconical such that the distal tip (36) is generally planar.

5. A nozzle (20) as claimed in any one of Claims 2 to 4, wherein the body (22) comprises a proximal shoulder (32) extending around the external surface of the body (22).

6. A nozzle (20) as claimed in Claim 5, wherein the proximal end (26) of the body (22) extends proximally from the proximal shoulder (32) and wherein the proximal end (26) of the body (22) tapers in a proximal direction from the proximal shoulder (32) towards the proximal end (26) along the longitudinal axis (24).

7. A nozzle (20) as claimed in Claim 6, wherein the proximal end (26) of the body (22) is frustoconical such that the proximal tip (36) is generally planar.

8. A nozzle (20) as claimed in Claim 7, wherein the inlet (21) is positioned on the proximal tip (36) and wherein the inlet (21) is concentrically aligned with the longitudinal axis (24).

9. A nozzle (20) as claimed in any one of Claims 6 to 8, wherein a central body portion (35) is defined between the distal shoulder (30) and the proximal shoulder (32).

10. A nozzle (20) as claimed in Claim 9, wherein the central body portion (35) tapers in a proximal direction along the longitudinal axis (24) from the distal shoulder (30) towards the proximal shoulder (32).

11. A nozzle (20) as claimed in Claim 9, wherein the central body portion (35) has generally parallel edges such that the cross sectional area of the central body portion (35) is substantially constant along the longitudinal axis (24) between the distal (30) and proximal (32) shoulders.

12. A nozzle (20) as claimed in any preceding claim, wherein the at least one exit gate (25) extends substantially perpendicularly from the passage (27) to an external surface of the distal end (28).

13. A nozzle (20) as claimed in any preceding claim, comprising four exit gates (25) positioned circumferentially around the distal end (28) of the body (22); and/or wherein the at least one exit gate (25) is generally square shape.

14. A syringe (100) for delivering blood plasma to a target treatment site (16) comprising a nozzle (20) as claimed in any preceding claim.

15. A syringe (100) as claimed in Claim 14, wherein a flexible tube (104) fluidly connects the nozzle (20) to the syringe (100).

## Patentansprüche

1. Düse (20) für eine Spritze (100) zum Verteilen von Blutplasma an einer Zielbehandlungsstelle (16) innerhalb des Mundes eines Patienten, wobei die Düse (20) Folgendes umfasst:
einen Körper (22) mit einem Durchgang (27), der sich durch den Körper (22) entlang einer Längsachse (24) des Körpers (22) erstreckt, wobei sich der Durchgang (27) von einem proximalen Ende (26) zu einem distalen Ende (28) des Körpers (22) erstreckt;
einen Einlass (21) zu dem Durchgang (27) an dem proximalen Ende (26) zum Aufnehmen von Blutplasma von der Spritze (100);
einen Auslass aus dem Durchgang (27) an dem distalen Ende (28) zum Verteilen von Blutplasma an der Zielbehandlungsstelle (16), wobei der Auslass eine Auslassöffnung (23) umfasst, die sich an einer distalen Spitze (36) der Düse (20) befindet; und
mindestens eine Austrittsöffnung (25), die sich von dem Durchgang (27) durch das distale Ende (28) des Körpers (22) erstreckt, **dadurch gekennzeichnet, dass** sich die mindestens eine Austrittsöffnung (25) von dem Durchgang (27) aus nach außen verjüngt.

2. Düse (20) nach Anspruch 1, wobei der Körper (22) eine distale Schulter (30) umfasst, die sich um eine Außenfläche des Körpers (22) erstreckt.

3. Düse (20) nach Anspruch 2, wobei sich das distale Ende (28) des Körpers (22) distal von der distalen Schulter (30) erstreckt und wobei sich das distale Ende (28) des Körpers (22) in einer distalen Richtung von der distalen Schulter (30) zu der distalen Spitze (36) entlang der Längsachse (24) verjüngt.

4. Düse (20) nach Anspruch 3, wobei das distale Ende (28) des Körpers (22) kegelstumpfförmig ist, sodass die distale Spitze (36) im Allgemeinen planar ist.

5. Düse (20) nach einem der Ansprüche 2 bis 4, wobei der Körper (22) eine proximale Schulter (32) umfasst, die sich um die Außenfläche des Körpers (22) erstreckt.

6. Düse (20) nach Anspruch 5, wobei sich das proximale Ende (26) des Körpers (22) proximal von der proximalen Schulter (32) erstreckt und wobei sich das proximale Ende (26) des Körpers (22) in einer proximalen Richtung von der proximalen Schulter (32) zu dem proximalen Ende (26) entlang der Längsachse (24) verjüngt.

7. Düse (20) nach Anspruch 6, wobei das proximale Ende (26) des Körpers (22) kegelstumpfförmig ist, sodass die proximale Spitze (36) im Allgemeinen planar ist.

8. Düse (20) nach Anspruch 7, wobei der Einlass (21) an der proximalen Spitze (36) angeordnet ist und wobei der Einlass (21) konzentrisch mit der Längsachse (24) ausgerichtet ist.

9. Düse (20) nach einem der Ansprüche 6 bis 8, wobei ein zentraler Körperabschnitt (35) zwischen der distalen Schulter (30) und der proximalen Schulter (32) definiert ist.

10. Düse (20) nach Anspruch 9, wobei sich der zentrale Körperabschnitt (35) in einer proximalen Richtung entlang der Längsachse (24) von der distalen Schulter (30) zu der proximalen Schulter (32) hin verjüngt.

11. Düse (20) nach Anspruch 9, wobei der zentrale Körperabschnitt (35) im Allgemeinen parallele Kanten aufweist, sodass die Querschnittsfläche des zentralen Körperabschnitts (35) im Wesentlichen konstant entlang der Längsachse (24) zwischen der distalen (30) und proximalen (32) Schulter ist.

12. Düse (20) nach einem der vorstehenden Ansprüche, wobei sich die mindestens eine Austrittsöffnung (25) im Wesentlichen senkrecht von dem Durchgang (27) zu einer Außenfläche des distalen Endes (28) erstreckt.

13. Düse (20) nach einem der vorstehenden Ansprüche, umfassend vier Austrittsöffnungen (25), die in Umfangsrichtung um das distale Ende (28) des Körpers (22) herum angeordnet sind; und/oder
wobei die mindestens eine Austrittsöffnung (25) im Allgemeinen eine quadratische Form aufweist.

14. Spritze (100) zum Abgeben von Blutplasma an eine Zielbehandlungsstelle (16), umfassend eine Düse (20) nach einem der vorstehenden Ansprüche.

15. Spritze (100) nach Anspruch 14, wobei ein flexibler Schlauch (104) die Düse (20) mit der Spritze (100) fluidisch verbindet.

## Revendications

1. Buse (20) pour une seringue (100) pour distribuer du plasma sanguin au niveau d'un site de traitement cible (16) à l'intérieur de la bouche d'un patient, la buse (20) comprenant :
un corps (22) ayant un passage (27) s'étendant à travers le corps (22) le long d'un axe longitudinal (24) du corps (22), le passage (27) s'étendant d'une extrémité proximale (26) à une extrémité distale (28) du corps (22) ;
une entrée (21) du passage (27) au niveau de l'extrémité proximale (26) pour recevoir le plasma sanguin à partir de la seringue (100) ;
une sortie du passage (27) au niveau de l'extrémité distale (28) pour distribuer du plasma sanguin au niveau du site de traitement cible (16), dans lequel la sortie comprend une ouverture de sortie (23) située sur une pointe distale (36) de la buse (20) ; et
au moins une porte de sortie (25) s'étendant à partir du passage (27) à travers l'extrémité distale (28) du corps (22), **caractérisée en ce que** l'au moins une porte de sortie (25) se rétrécit vers l'extérieur à partir du passage (27).

2. Buse (20) selon la revendication 1, dans laquelle le corps (22) comprend un épaulement distal (30) s'étendant autour d'une surface externe du corps (22).

3. Buse (20) selon la revendication 2, dans laquelle l'extrémité distale (28) du corps (22) s'étend de manière distale à partir de l'épaulement distal (30) et dans laquelle l'extrémité distale (28) du corps (22) se rétrécit dans une direction distale à partir de l'épaulement distal (30) vers l'extrémité distale (36) le long de l'axe longitudinal (24).

4. Buse (20) selon la revendication 3, dans laquelle l'extrémité distale (28) du corps (22) est tronconique de sorte que la pointe distale (36) est généralement plane.

5. Buse (20) selon l'une quelconque des revendications 2 à 4, dans laquelle le corps (22) comprend un épaulement proximal (32) s'étendant autour de la surface externe du corps (22).

6. Buse (20) selon la revendication 5, dans laquelle l'extrémité proximale (26) du corps (22) s'étend de manière proximale à partir de l'épaulement proximal (32) et dans laquelle l'extrémité proximale (26) du corps (22) se rétrécit dans une direction proximale à partir de l'épaulement proximal (32) vers l'extrémité proximale (26) le long de l'axe longitudinal (24).

7. Buse (20) selon la revendication 6, dans laquelle l'extrémité proximale (26) du corps (22) est tronconique de sorte que la pointe proximale (36) est généralement plane.

8. Buse (20) selon la revendication 7, dans laquelle l'entrée (21) est positionnée sur la pointe proximale (36) et dans laquelle l'entrée (21) est alignée de manière concentrique avec l'axe longitudinal (24).

9. Buse (20) selon l'une quelconque des revendications 6 à 8, dans laquelle une partie de corps centrale (35) est définie entre l'épaulement distal (30) et l'épaulement proximal (32).

10. Buse (20) selon la revendication 9, dans laquelle la partie de corps centrale (35) se rétrécit dans une direction proximale le long de l'axe longitudinal (24) de l'épaulement distal (30) vers l'épaulement proximal (32).

11. Buse (20) selon la revendication 9, dans laquelle la partie de corps centrale (35) a des bords généralement parallèles de sorte que la zone de section transversale de la partie de corps central (35) est sensiblement constante le long de l'axe longitudinal (24) entre les épaulements distal (30) et proximal (32).

12. Buse (20) selon l'une quelconque des revendications précédentes, dans laquelle l'au moins une porte de sortie (25) s'étend sensiblement perpendiculairement du passage (27) à une surface externe de l'extrémité distale (28).

13. Buse (20) selon l'une quelconque des revendications précédentes, comprenant quatre portes de sortie (25) positionnées circonférentiellement autour de l'extrémité distale (28) du corps (22) ; et/ou
dans lequel l'au moins une porte de sortie (25) est généralement de forme carrée.

14. Seringue (100) pour distribuer du plasma sanguin à un site de traitement cible (16) comprenant une buse (20) selon l'une quelconque des revendications précédentes.

15. Seringue (100) selon la revendication 14, dans laquelle un tube flexible (104) relie de manière fluidique la buse (20) à la seringue (100).
